# FASCICULE DE BREVET EUROPEEN

(11) **EP 3 308 850 B1**
(45) Date de publication et mention de la délivrance du brevet: **22.05.2019**
(21) Numéro de dépôt: 17195135.3
(22) Date de dépôt: 06.10.2017
(51) Int. Cl.: B01F 13/08, C12M 1/00, C12M 1/02

(54) **PREPARATEUR DE MILIEU DE CULTURE COMPRENANT UNE CUVE DOTEE D'UN AGITATEUR CENTRAL MIS EN ROTATION PAR UN ANNEAU MAGNETIQUE**
VORBEREITER EINES KULTURMILIEUS, DER EINE SCHALE UMFASST, DIE MIT EINEM ZENTRALEN SCHÜTTLER AUSGESTATTET IST UND DURCH EINEN MAGNETISCHEN RING IN ROTATION VERSETZT WIRD
DEVICE FOR PREPARING A CULTURE MEDIUM COMPRISING A VESSEL PROVIDED WITH A CENTRAL AGITATOR ROTATED BY A MAGNETIC RING

(30) Priorité: 12.10.2016 FR 1659865
(43) Date de publication de la demande: 18.04.2018
(73) Titulaire: Alliance Bio Expertise, 35480 Guipry (FR)
(72) Inventeur: LE SAUX, Philippe, 44130 BLAIN (FR); GILET, Morgan, 22350 CAULNES (FR)
(74) Mandataire: Vidon Brevets & Stratégie

(56) Documents cités:
- EP-A2- 0 257 750
- EP-A2- 0 259 002
- WO-A1-94/06553

## Description

### 1. Domaine de l'invention

L'invention concerne un dispositif pour maintenir une solution dans un milieu régulé en température en l'agitant en permanence. L'invention s'applique plus précisément pour un dispositif disposant d'une cuve pour la préparation de la solution dotée d'un agitateur animé en rotation par un mécanisme magnétique.

### 2. Art antérieur

Dans le monde industriel, tout produit commercialisé doit répondre aux normes en vigueur, en particulier les produits alimentaires sur lesquels s'appliquent des normes sanitaires strictes. Dans le domaine de l'agro-alimentaire, ces normes imposent notamment une quantité maximale de micro-organismes tels que des bactéries, dans un volume donné de produit. Pour vérifier la présence de ces micro-organismes et déterminer leur quantité, on a l'habitude d'effectuer des prélèvements de nourritures, et d'étudier l'évolution des micro-organismes dans un milieu qui favorisent grandement leur prolifération.

Pour cela, des masses précises sont prélevées dans des lots de produits afin de produire des échantillons, qui sont ensuite placés dans des boites de Pétri. La boîte contient préalablement une solution appelée gélose qui favorise la prolifération de ces micro-organismes lorsque ladite boite est placée pendant une certaine durée dans une enceinte régulée selon une température et selon un pression bien précises. Les boites sont ouvertes à des moments différents, typiquement plusieurs jours et soumises à des tests pour mesurer la densité de micro-organismes présente dans la boite et son évolution au fil du temps. Si la densité est inférieure à un certain seuil, alors le lot dont est extrait l'échantillon est considéré propre à la consommation.

La production de gélose doit s'effectuer dans un milieu stérile pour réaliser des échantillons non pollués. La production s'effectue dans des autoclaves régulés en température et en pression. L'enceinte de l'autoclave comporte une cuve dotée d'une double enveloppe qui communique le froid ou la chaleur à la solution contenue à l'intérieur. Mais cette enveloppe occupe une large portion en hauteur de la cuve, de sorte que si le contenu est en faible quantité et se concentre en bas de la cuve, le serpentin chauffe et/ou refroidit inutilement les parois, ce qui génère un manque d'homogénéité de la température.

Pour limiter ce problème, les autoclaves actuels disposent d'un agitateur entrainé par un moteur, équipé d'un système magnétique, situé en dessous de la cuve. Cet agitateur est généralement constitué de pales métalliques, équipés d'aimants, en rotation autour d'un axe fixé en fond de cuve. Cette disposition entraîne une augmentation de la hauteur de l'appareil pour laisser la place à la mécanique d'entrainement de l'agitateur sous la cuve. De plus, les liaisons mécaniques et magnétiques situées sous la cuve ne permettent pas de chauffer ou refroidir le fond de la cuve à l'aide d'un élément chauffant et d'un dispositif de refroidissement, mais aussi d'avoir un prélèvement central du milieu de culture, à travers un tube. Le nettoyage est une étape sensible car il est important de rendre la cuve parfaitement propre et stérile avant de commencer une autre production.

Le document WO 94/06553 publié le 31 Mars 1994, divulgue un autoclave et un gobelet amovible placé dans une cuve. Le gobelet est muni d'un compartiment de mélange adapté pour recueillir et mélanger des produits à traiter, et d'un compartiment d'agitation à l'aide d'un objet magnétique entrainé en rotation par un jeu d'aimants placé sous l'autoclave. Cet autoclave ne mélange réellement le produit qu'en partie basse de la cuve, là ou l'agitateur se situe.

Il existe donc un réel besoin d'un préparateur de solution dont la cuve est facile à nettoyer en utilisant un chiffon pour essuyer l'intérieur et en utilisant un liquide de nettoyage que l'on peut facilement évacuer.

### 3. Objectifs de l'invention

La présente invention propose un nouveau modèle de préparateur de milieu de culture qui possède une cuve présentant le moins d'aspérités à l'intérieur tout en étant doté d'un agitateur, et un dispositif de stérilisation efficace.

### 4. Exposé de l'invention

L'invention a notamment pour objet un préparateur de milieu de culture comportant une cuve de forme cylindrique se terminant en partie basse par une calotte sensiblement hémisphérique, un couvercle et un agitateur placé dans la cuve. L'agitateur comporte un tube vertical placé au centre de la cuve et une pluralité de pales mises en rotation autour dudit tube et des masses magnétiques en périphérie de façon à les placer à proximité de la paroi intérieure de la cuve. Le préparateur comporte en outre un anneau magnétique en rotation autour de la paroi intérieure à la même hauteur que les masses magnétiques, lesdits aimants attirant lesdites masses magnétiques, l'anneau magnétique étant animé en rotation par un moteur.

De cette façon, l'agitateur est entraîné en rotation sans nécessiter d'éléments mécaniques placés à l'intérieur de la cuve, l'absence de tels éléments facilitant le nettoyage.

Selon un mode particulier de réalisation, le préparateur de milieu de culture comporte en outre une première cavité chauffante au contact avec la paroi intérieure et autour de la partie cylindrique et une seconde cavité chauffante placée au contact avec la paroi intérieure et sous la cuve, l'anneau magnétique étant placé dans l'espace laissé libre entre les deux cavités. De cette manière, le mécanisme d'entraînement de l'agitateur ne se trouve pas sous la cuve, ce qui permet de placer des moyens de chauffage à cet endroit, et la hauteur de l'appareil est plus limitée.

Selon un autre mode de réalisation, chacune des cavités comporte au moins une vanne d'entrée et une vanne de sortie permettant de créer un courant de fluide traversant ladite cavité. De cette manière, il est possible de faire passer dans ces cavités un courant de fluide pour refroidir, mais aussi éventuellement réchauffer, plus rapidement le contenu de la cuve.

Selon un autre mode de réalisation, l'extrémité haute du tube de l'agitateur passe à travers un orifice du couvercle et en ce que l'extrémité basse du tube se glisse à l'intérieur d'un insert situé au centre de la calotte hémisphérique, l'orifice du couvercle étant situé à la verticale de l'axe central de la cuve lorsque le couvercle est abaissé de façon à centrer la partie haute de l'agitateur. De cette manière, en ouvrant le couvercle les éléments de l'agitateur sont désolidarisés de la cuve et peuvent très facilement être enlevés, pour le nettoyage par exemple.

Selon un autre mode de réalisation, la calotte sensiblement hémisphérique est percée en son centre d'un trou d'évacuation, l'extrémité inférieure du tube constituant un bouchon pour obstruer ledit trou. De cette manière, l'évacuation du liquide de nettoyage est facilitée.

Selon un autre mode de réalisation, le tube est creux et ouvert à ses deux extrémités de façon à extraire par aspiration le contenu liquide de la cuve. De cette manière, il est possible d'aspirer le contenu de la cuve au cours de la préparation sans gêner le mouvement de l'agitateur.

Selon un autre mode de réalisation, le préparateur de milieu de culture comporte une centrale de régulation contrôlant la température d'éléments chauffant placés au contact des cavités, ladite centrale de régulation activant uniquement l'élément chauffant placé au contact de la seconde cavité lorsque le produit placé à l'intérieur de la cuve ne dépasse par une hauteur déterminée. De cette manière, les moyens de chauffage peuvent s'adapter à la quantité de liquide à chauffer dans la cuve.

### 5. Liste des figures

D'autres caractéristiques et avantages de l'invention apparaîtront maintenant avec plus de détails dans le cadre de la description qui suit d'exemples de réalisation donnés à titre illustratif et non-limitatif en se référant aux figures annexées qui représentent :
- la figure 1 présente une vue en coupe verticale d'un préparateur de milieu de culture selon un exemple de réalisation de l'invention,
- la figure 2 présente un schéma vu du dessus du préparateur au niveau de l'anneau d'entrainement de l'agitateur selon un exemple de réalisation,
- la figure 3 présente une vue en coupe verticale de la partie basse du préparateur selon un perfectionnement de l'invention,
- la figure 4 présente le plan de la partie basse découpée d'un prototype de préparateur de milieu de culture selon un exemple de réalisation,
- la figure 5 présente le plan de la partie basse d'un prototype de préparateur de milieu de culture selon un exemple de réalisation,
- la figure 6 présente le plan de la partie haute et du couvercle d'un prototype de préparateur de milieu de culture selon un exemple de réalisation.

### 6. Description d'un mode de réalisation de l'invention

### 6.1 Principe général

L'invention consiste en un préparateur de milieu de culture comportant une cuve de forme cylindrique se terminant en partie basse par une calotte sensiblement hémisphérique, un couvercle et un agitateur placé dans la cuve. L'agitateur comporte un tube vertical placé au centre de la cuve, une pluralité de pales mises en rotation autour dudit tube et des masses magnétiques en périphérie de façon à les placer à proximité de la paroi intérieure de la cuve. Le préparateur comporte en outre un anneau magnétique en rotation autour de la paroi intérieure à la même hauteur que les masses magnétiques, lesdits aimants attirant lesdites masses magnétiques, l'anneau magnétique étant animé en rotation par un moteur. De cette façon, l'agitateur est entraîné en rotation sans nécessiter d'éléments mécaniques placés à l'intérieur de la cuve, l'absence de tels éléments facilitant le nettoyage.

### 6.2 Description d'un mode de réalisation

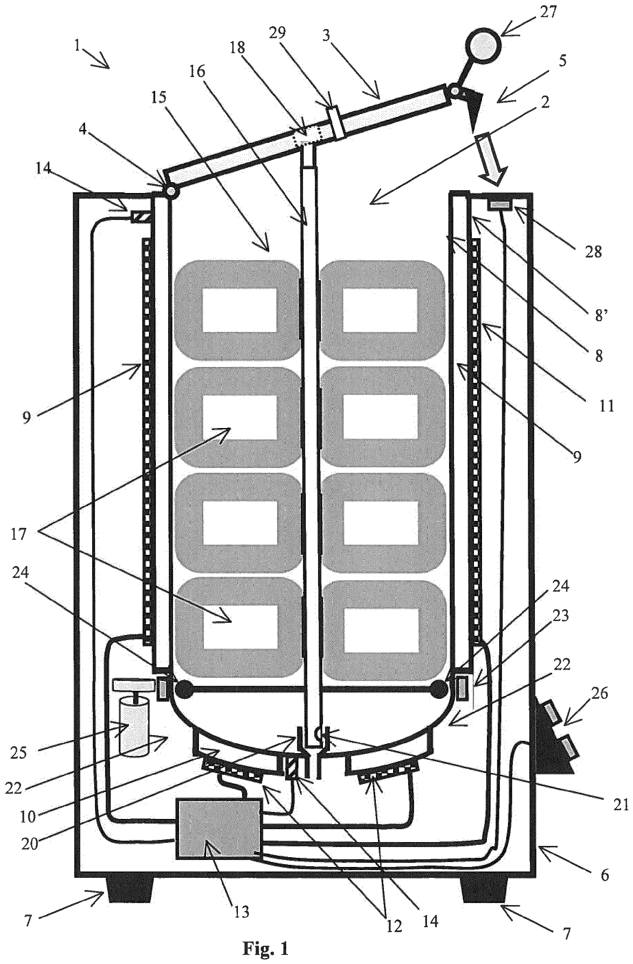

La **FIG.1** illustre un préparateur de milieu de culture 1. Cet appareil comporte une cuve 2 de forme cylindrique avec un fond en forme de calotte sensiblement hémisphérique et présentant une ouverture circulaire orientée vers le haut. La cuve 2, d'une capacité de 30 litres par exemple, est hermétiquement fermée par un couvercle 3 pivotant comportant au moins deux charnières 4 sur un coté et au moins deux verrous 5 sur le coté opposé, les verrous venant s'accrocher sur les axes fixés au bâti 6 de l'appareil. Une fois la cuve fermée, l'intérieur peut être mis en pression avec des valeurs de 1 à 2 bars. La sécurité impose que chaque verrou peut indépendamment de l'autre maintenir la cuve en position hermétiquement fermée. La cuve est incorporée dans un bâti 6 posé sur des pieds 7.

L'enveloppe de la cuve 2, fabriquée par exemple en acier inoxydable, est constituée d'une paroi intérieure 8 et d'une paroi extérieure 8'. La cuve est de forme cylindrique avec un fond sensiblement sphérique. La paroi extérieure 8' s'interrompt au niveau de la partie basse du flanc cylindrique et reprend pour recouvrir une partie du fond de la cuve en formant un plateau sensiblement hémisphérique et creux. La double enveloppe de la cuve réalise ainsi deux cavités l'une 9 en partie haute et l'autre 10 au fond de la cuve, ces cavités étant destinée à contenir un liquide, de l'eau du réseau par exemple. Une fois les cavités remplies, la double enveloppe 8 et 8' est isolée par la fermeture d'électrovannes d'entrée et de sortie (non représentées sur la **Fig.1**).

La cuve dispose de deux moyens de chauffage, le premier 11 est positionné autour de la paroi extérieure 8' au contact de la cavité 9, le second 12 est placé sous la cuve au contact de la paroi extérieure 8' de la cavité 10. Selon un exemple préféré de réalisation, ces moyens de chauffage sont réalisés par des résistances électriques. On peut noter que la résistance du fond de cuve est le moyen le plus efficace pour monter rapidement en température. Le contrôle de la température du liquide s'effectue par une centrale de régulation 13. La montée en température du contenant de la cuve s'effectue par le contact avec la paroi intérieure 8.

Selon un perfectionnement, plusieurs capteurs 14 sont placés au contact de la paroi intérieure de la cuve afin de mesurer la température à cet endroit, l'un étant placé en haut de la cuve et l'autre au niveau du fond de la cuve. Les capteurs 14 sont reliés à la centrale de régulation 13 qui, en fonction des valeurs mesurées, commande la montée et la descente en température du liquide caloporteur circulant dans la cavité inférieure 10 et/ou la cavité supérieure 9.

Un agitateur vertical 15 est placé au centre de la cuve, il comporte un axe vertical constitué d'un tube droit creux 16, d'un diamètre de 17 à 18 centimètres de préférence, et ouvert aux deux extrémités. L'agitateur comporte également une pluralité de pales 17 formant des surfaces verticales ajourées regroupées selon plusieurs plans verticaux, par exemple 2 plans s'étendant axialement par rapport au tube 16 et se coupant à angle droit. Les pales sont solidarisées entre elles et constituent un ensemble monobloc qui tourne autour de l'axe à l'aide d'au moins deux paliers placés en haut et en bas. Chaque groupe de pales sur un rayon possède son symétrique de l'autre coté de l'axe vertical, de cette manière l'agitateur est équilibré et son centre de gravité se situe sur l'axe central du tube droit 16. Le tube droit traverse le couvercle 3 par un orifice circulaire 18 qui se situe à la verticale de l'axe central de la cuve lorsque le couvercle est abaissé. De cette façon, l'agitateur est centré verticalement au milieu de la cuve 2. La largeur de l'agitateur définie par les groupes de pales est inférieure de quelques millimètres au diamètre intérieur de la cuve. De cette façon, les pales de l'agitateur frôlent les parois de la cuve et assure un mélange homogène, en mélangeant les composants et en permettant une température homogène. Lorsque l'opérateur referme le couvercle l'extrémité supérieure du tube 16 ressort naturellement par l'orifice 18. La section inférieure de cet orifice est tronconique et se rétrécit vers le haut pour améliorer le centrage et éviter que les arêtes externes des pales 17 ne viennent toucher les parois intérieures de la cuve.

La base du tube est engagée dans un insert creux démontable 20, ajourée de 4 trous cylindriques à sa base. Ces trous permettent la sortie du liquide contenu dans la cuve à travers au moins un trou 21 pratiqué tout en bas du tube creux 16. Cette disposition permet d'aspirer le contenu de la cuve jusqu'à un niveau très bas, ce qui réduit au minimum le volume mort de la cuve. L'insert 20 est lui-même engagé dans le trou de vidange de la cuve qui le maintient en position vertical, dans l'axe de la cuve, sans jeu, grâce à un joint torique qui annule ce jeu. Cet insert est équipé d'un joint à lèvre au niveau de la partie la plus large, en contact avec la cuve, assurant ainsi une étanchéité parfaite grâce à la pression de l'agitateur. De cette manière, et selon un mode préféré de réalisation, l'extrémité du tube sert de bouchon et le fait de l'ôter permet au contenu de s'écouler par un tuyau (non représenté sur la Fig. 1). De cette manière, le nettoyage s'effectue à grande eau, le liquide souillé s'écoulant par gravitation à travers un orifice d'évacuation situé dans la partie la plus basse de la cuve.

L'insert comporte donc plusieurs avantages en servant de :
- bouchon de vidange,
- axe de rotation pour l'agitateur,
- guide pour le tube de prélèvement,
- embout de prélèvement du tube.

Cette disposition évite que la fermeture de l'orifice d'évacuation s'effectue par une vanne déportée sous la cuve, ou à l'extérieur de la cuve, ce qui entraîne la présence d'un volume dans un tuyau qui ne peut être chauffé par les résistances électrique et donc ne peut être efficacement stérilisé. Le fait de placer l'insert servant de bouchon au fond de la cuve assure la stérilisation du volume complet de la cuve, car l'insert servant de bouchon est lui-même mis à la température de 121°C.

Comme on le voit sur la **Fig. 1**, l'agitateur 12 est animé en rotation autour de son axe vertical, et est centré au milieu de la cuve par l'insert 16 en partie basse et l'orifice 15 dans la couvercle.

L'extraction du contenu liquide de la cuve peut s'effectuer par un trou 17 pratiqué en bas du tube 13. L'extrémité supérieure du tube est reliée par un joint rotatif étanche à un tuyau lui-même relié à une pompe (non représentée sur la **Fig. 1**). La pompe génère un vide dans le tuyau qui aspire le contenu et le verse dans des récipients, des boites de Pétri par exemple. Le fait d'utiliser le tube au centre de l'agitateur comme moyen d'extraction du contenu de la cuve permet d'éviter des bouches de sortie de la cuve qui pourraient s'encrasser et qui, de toutes les façons, seraient difficiles à nettoyer. Le nettoyage du tube est facilité par le fait qu'il est simplement enfilé dans l'insert, il suffit donc d'exercer une simple traction verticale pour l'enlever de la cuve. Le démontage de l'agitateur et de l'insert s'effectue de la même manière, après avoir retiré le tube.

Comme on peut le voir sur la **Fig.1**, les cavités 9 et 10 sont séparées d'un espace en forme d'anneau hémisphérique, la cuve en contact avec cet espace est dépourvue de paroi extérieure 8'. Cet espace est mis à profit pour y installer le mécanisme d'entraînement en rotation de l'agitateur 15.

Selon un mode préféré de réalisation, l'espace 22 situé entre les cavités 9 et 10 comporte un anneau 23 capable de tourner tout autour de la partie basse de la cuve. L'anneau comporte une pluralité d'aimants disposés à intervalles réguliers. Les aimants attirent un nombre égal de masses magnétiques 24 placées à l'extrémité des pales à la même hauteur que l'anneau 23 et disposées radialement par rapport au tube droit 13. La géométrie du placement des aimants et des masses magnétiques de l'agitateur est conçue de façon que ces éléments se situent à une distance minimale en étant séparés par la paroi intérieure de la cuve. Les masses magnétiques 18 peuvent aussi être réalisées par les pales elles-mêmes, celles-ci seront alors métalliques. De petits galets équipés de roulement à billes, sont placés dans l'espace au fond de la gorge 16 facilitent la rotation de l'anneau. Un moteur 25 commandé par la centrale de régulation 13 entraîne une courroie en rotation, celle-ci entourant l'anneau et animant son mouvement. La rotation de l'anneau entraîne celle de l'agitateur, la vitesse de rotation dépendant de celle du moteur 19. Le couplage magnétique évite le percement de la cuve pour le passage d'un arbre d'entrainement, ce qui facilite son nettoyage. La forme cylindrique avec un fond sensiblement hémisphérique de la cuve et le fait de ne présenter aucune aspérité venant gêner le passage d'une éponge ou d'un chiffon contribue aussi à un nettoyage facile.

Selon une variante de réalisation, le moteur 25 entraîne une roue à friction qui est au contact avec la surface externe de l'anneau 23.

Le fait que le mécanisme d'entrainement en rotation de l'agitateur se situe en-dessous et en périphérie du fond de la cuve a pour avantage de libérer de la place pour y placer le moyen de chauffage 12 et un orifice d'évacuation au fond de la cuve.

Les réglages de l'appareil sont introduits par une Interface Homme Machine (IHM) qui peut se présenter sous la forme d'un clavier 26 avec des boutons et des afficheurs, ou sous la forme d'un clavier tactile. Une acquisition par la voix avec un moteur de reconnaissance vocale est également possible comme moyen d'acquisition de commandes. L'IHM peut comporter un buzzer ou un haut-parleur pour émettre des sons, notamment des signaux d'alarme.

Le couvercle 3 est solidement fermé par des verrous 5 qui s'insèrent dans des gorges solidaires du bâti 6. Les verrous se prolongent vers le haut par une poignée 27, le tout pivotant autour d'un axe horizontal. Un capteur 28 contrôle en permanence la position du verrou et interrompt immédiatement la rotation de l'agitateur lorsque la poignée est manoeuvrée. Avantageusement, un verrou magnétique bloque l'ouverture en position fermée tant que l'agitateur tourne.

Selon un perfectionnement, le couvercle dispose d'une vanne de communication 29 avec l'extérieur de l'appareil. Cette vanne permet notamment l'entrée d'air à l'intérieur de la cuve après passage à travers un filtre. Cette entrée est protégée par un filtre à particules en amont (cage de coton cardé) et par un filtre disque de 0.2*µ*M en aval, afin d'éviter toute contamination du milieu préparé, à travers cette électrovanne. La vanne 29 est ouverte à la fin de la préparation du contenu afin d'équilibrer les pressions avant d'ouvrir le couvercle.

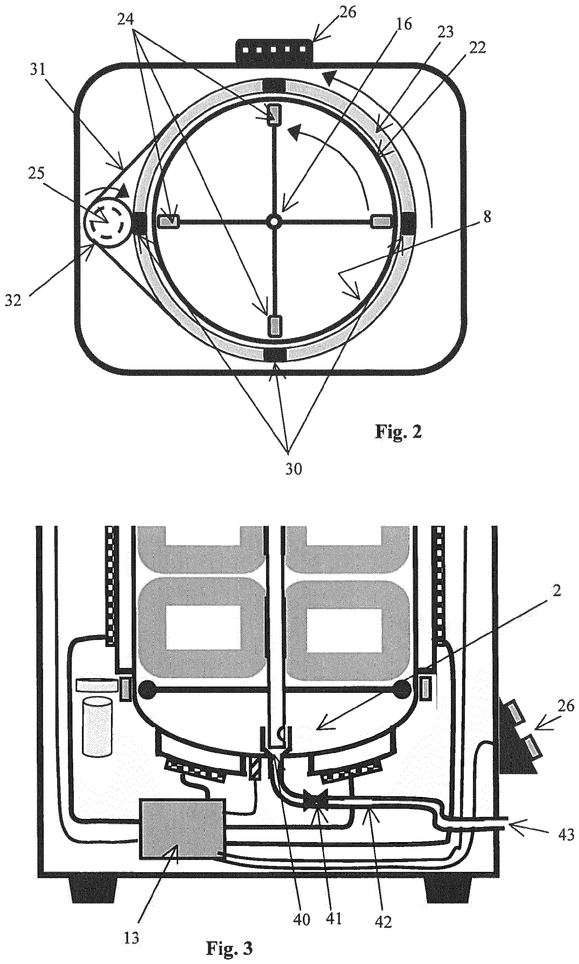

La **FIG.2** présente une coupe horizontale du préparateur de milieu de culture 1 au niveau de l'espace 22 laissé libre entre les deux cavités chauffantes 9 et 10. L'espace libre qui se prolonge en profondeur jusqu'à l'enveloppe interne de la cuve 8, comporte un anneau circulaire 23 conçu pour tourner à l'intérieur de l'espace libre 22 à une distance la plus proche possible de l'enveloppe interne de la cuve 8. Des petits galets assurent le guidage en rotation de l'anneau circulaire 23. L'anneau 23 dispose à intervalle régulier d'aimants 30, au nombre de 4 sur la **Fig. 2**, (ce nombre étant donné qu'à titre d'exemple). Chaque aimant étant placé par rapport à ses voisins à 90 ° de rotation l'un de l'autre. Les aimants 30 attirent un nombre égal de masses magnétiques 24 placées en périphérie de l'agitateur, le plus près possible de l'enveloppe interne 8. Les écarts angulaires des masses magnétiques sont égaux à ceux des aimants 22 de l'anneau 17, de sorte que l'agitateur tourne naturellement pour réduire la distance entre les masses et les aimants. Les caractéristiques mécaniques des aimants 22 et des masses sont choisies pour que les forces magnétiques s'équilibrent parfaitement, permettant ainsi à l'agitateur de se tenir dans une position strictement verticale lors de la rotation, son axe se confondant avec l'axe central de la cuve.

L'anneau est animé en rotation par une courroie 31, elle-même entrainée par une roue à friction 32 fixée sur l'axe d'un moteur 25 (vue par transparence sur la **Fig. 2**). La rotation de l'anneau 23 entraînant la rotation de l'agitateur selon une vitesse angulaire contrôlée par la centrale de régulation 13. La position du moyen d'entrainement de l'agitateur qui se situe autour de la cuve et pas en dessous, permet de diminuer la hauteur de l'appareil.

La centrale de régulation 13 reçoit des commandes introduites au niveau de l'IHM 26. Le préparateur saisit la quantité de produit à l'état liquide en terme de volume, et la centrale de régulation détermine en fonction de ce volume, la hauteur de produit dans la cuve 2. Si cette hauteur ne dépasse pas une hauteur déterminée comprise entre ¼ et ¾ de la hauteur totale de la cuve, la résistance 11 ceinturant la cuve n'est pas utilisée, et la centrale de régulation contrôle la température en utilisant uniquement la résistance 12 du fond de cuve. De cette façon, l'énergie est économisée puisqu'un seul moyen de chauffage est utilisé, et la régulation ne s'exerce pas sur des zones ne contenant pas de produit, ce qui évite la formation de point chaud ou froid et favorise une meilleure homogénéité de la température.

Selon un perfectionnement illustré par la **Fig. 3**, le fond de la cuve 2 comporte un orifice d'évacuation 40 dans sa partie la plus basse. Cet orifice se prolonge par un tuyau 42 et une vanne manuelle 41. Le tuyau 42 se prolonge après l'électrovanne et passe à travers le bâti pour sortir à l'extérieur par une bouche 43. Selon une variante de réalisation, l'électrovanne 41 peut être commandée par la centrale de régulation 13 et l'IHM 26, dans le cadre par exemple d'un cycle de nettoyage automatique.

Selon un exemple de fonctionnement, le contenu de la cuve est porté à 121°C pendant une durée de 15 minutes, et ensuite 47 °C qui est la température juste supérieure à celle limite de gélification. Cette descente de température est accélérée en ouvrant une vanne d'entrée et une vanne de sortie pour chaque cavité 9 et 10 et en injectant de l'eau froide venant du réseau (ou éventuellement d'un groupe frigorifique) pour créer un courant de liquide dans la cavité de la paroi de la cuve, et ainsi refroidir le contenu.

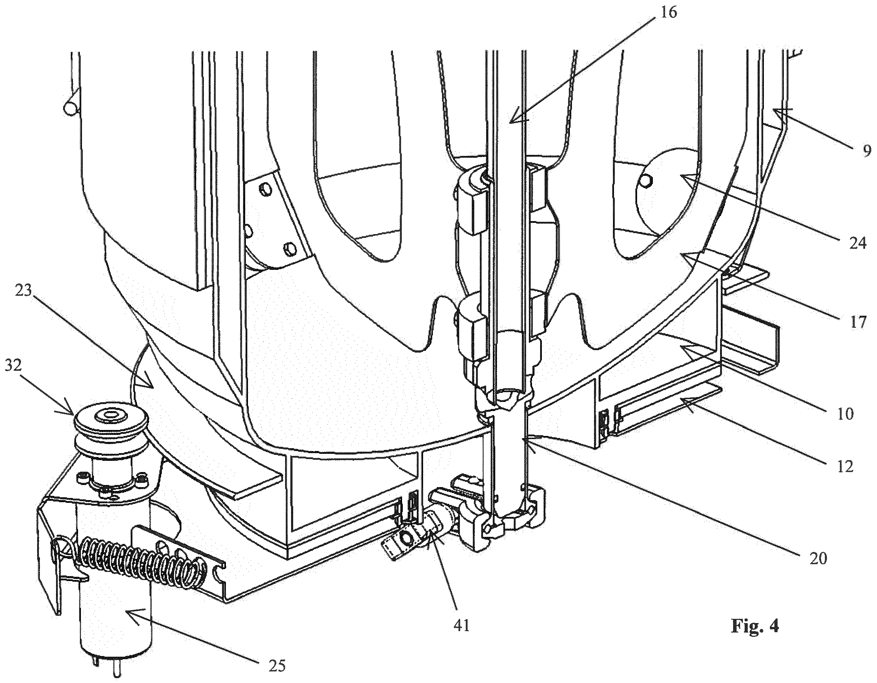

La **Fig. 4** présente le plan de la partie basse découpée d'un prototype de préparateur de milieu de culture selon un exemple de réalisation. Ce plan montre notamment la cavité 9 entourant le bas de la cuve et la cavité 10 située au-dessous du fond de la cuve, la résistance électrique 12 au contact de la cavité 10, les pales 17 de l'agitateur situées symétriquement autour de l'axe creux 16 centré en partie basse par l'insert 20, le moteur 25 et la poulie 32 entraînant en rotation l'anneau magnétique 23. L'insert se termine en partie basse par une sortie de liquide fermée par une vanne manuelle 41.

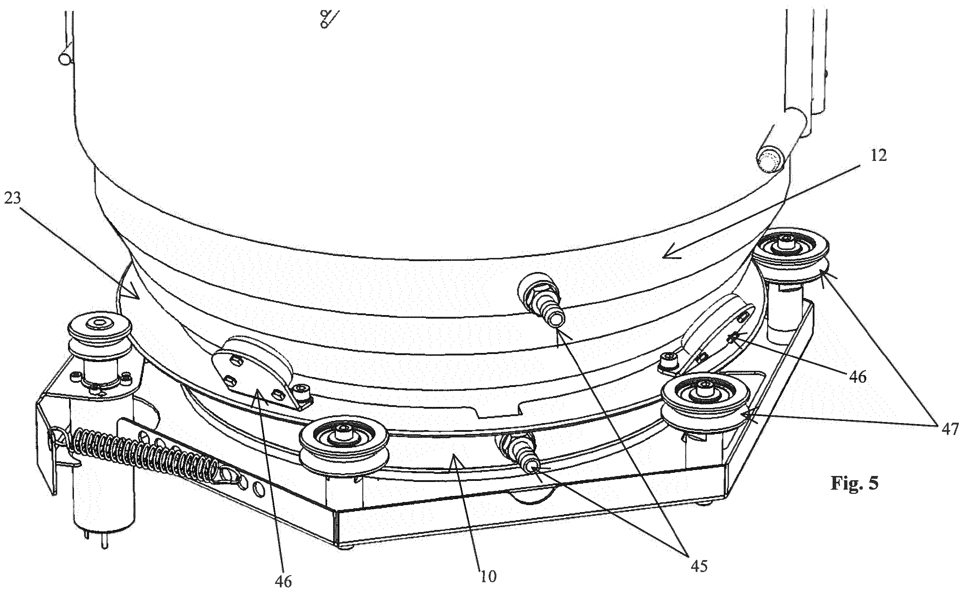

La **Fig. 5** présente le plan de la partie basse d'un prototype de préparateur de milieu de culture selon un exemple de réalisation. En plus de la figure précédente, on peut voir la position de valves d'entrée 45 et de sortie du liquide présent dans les cavités 9 et 10. Des tuyaux sont raccordés à ces valves pour transporter un liquide régulé en température, ou simplement de l'eau provenant du réseau pour refroidir rapidement le contenu de l'appareil. Cette figure montre également les aimants 46 montés sur l'anneau magnétique 23 ainsi que les galets 47 guidant la rotation de cet anneau. Sur cette figure, l'anneau magnétique est entrainé par une friction directe sur une poulie montée sur le moteur.

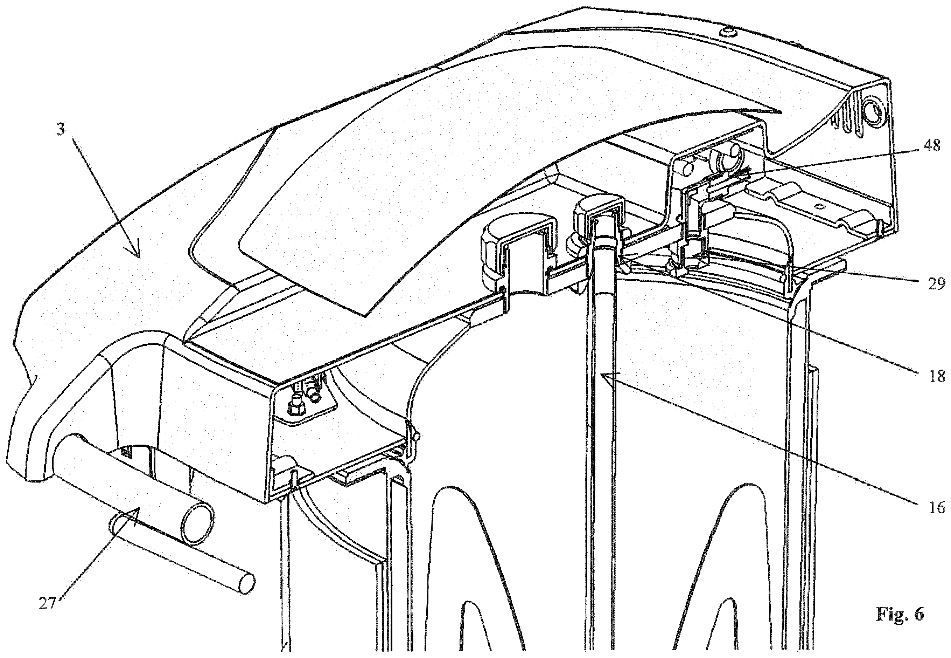

La **Fig. 6** présente le plan de la partie haute et du couvercle 3 d'un prototype de préparateur de milieu de culture selon un exemple de réalisation. Ce plan montre notamment l'orifice 18 de passage du tube creux 16 de l'agitateur, ainsi que la vanne de communication 29 permettant de faire communiquer l'intérieur de la cuve avec l'air ambiant. Cette vanne se prolonge vers la droite sur la figure par le filtre 48.

Bien que la présente invention ait été décrite en référence aux modes de réalisation particuliers illustrés, celle-ci n'est nullement limitée par ces modes de réalisation, mais ne l'est que par la teneur des revendications annexées ci-après. On notera que des changements ou des modifications pourront être apportés par l'Homme du métier.

## Revendications

1. Préparateur de milieu de culture (1) comportant une cuve (2) de forme cylindrique se terminant en partie basse par une calotte sensiblement hémisphérique, un couvercle (3) et un agitateur (15) placé dans la cuve (2), ledit agitateur comportant un tube (16) vertical placé au centre de la cuve et une pluralité de pales (17) mises en rotation autour dudit tube (16) ;
**caractérisé en ce que** l'agitateur comporte des masses magnétiques (24) en périphérie de façon à les placer à proximité de la paroi intérieure (8) de la cuve, le préparateur comportant en outre un anneau magnétique (23) en rotation autour de la paroi intérieure (8) à la même hauteur que les masses magnétiques (24), lesdits aimants attirant lesdites masses magnétiques, l'anneau magnétique étant animé en rotation par un moteur (25), ledit préparateur comportant de moyens de chauffage de la paroi ou du fond de la cuve pour réguler en température le contenu de ladite cuve.

2. Préparateur de milieu de culture selon la revendication 1, **caractérisé en ce qu'**il comporte en outre une première cavité chauffante (9) au contact avec la paroi intérieure (8) et autour de la partie cylindrique et une seconde cavité chauffante (10) placée au contact avec la paroi intérieure (8) et sous la cuve, l'anneau magnétique (23) étant placé dans l'espace (22) laissé libre entre les deux cavités.

3. Préparateur de milieu de culture selon la revendication 2, **caractérisé en ce que** chacune des cavités (9, 10) comporte au moins une vanne d'entrée et une vanne de sortie permettant de créer un courant de fluide traversant ladite cavité.

4. Préparateur de milieu de culture selon l'une quelconque des revendications précédentes, **caractérisé en ce que** l'extrémité haute du tube (16) de l'agitateur passe à travers un orifice (18) du couvercle (3) et **en ce que** l'extrémité basse du tube (16) se glisse à l'intérieur d'un insert (20) situé au centre de la calotte hémisphérique, l'orifice (18) du couvercle étant situé à la verticale de l'axe central de la cuve (3) lorsque le couvercle est abaissé de façon à centrer la partie haute de l'agitateur.

5. Préparateur de milieu de culture selon la revendication 4, **caractérisé en ce que** la calotte sensiblement hémisphérique est percée en son centre d'un trou d'évacuation, l'extrémité inférieure du tube constituant un bouchon pour obstruer ledit trou.

6. Préparateur de milieu de culture selon l'une quelconque des revendications précédentes, **caractérisé en ce que** le tube (16) est creux et ouvert à ses deux extrémités de façon à extraire par aspiration le contenu liquide de la cuve.

7. Préparateur de milieu de culture selon l'une quelconque des revendications précédentes sous la dépendance de la revendication 2, **caractérisé en ce qu'**il comporte une centrale de régulation (13) contrôlant la température d'éléments chauffant placés au contact des cavités (9, 10), ladite centrale de régulation (13) activant uniquement l'élément chauffant (12) placé au contact de la seconde cavité (10) lorsque le produit placé à l'intérieur de la cuve ne dépasse par une hauteur déterminée.

## Patentansprüche

1. Zubereiter eines Kulturmediums (1), umfassend eine Wanne (2) von zylindrischer Form, die im unteren Teil mit einer im Wesentlichen halbkugelförmigen Kalotte abschließt, einen Deckel (3) und ein Rührwerk (15), das in der Wanne (2) angeordnet ist, wobei das Rührwerk ein vertikales Rohr (16), das in der Mitte der Wanne angeordnet ist, und eine Vielzahl von Schaufeln (17) umfasst, die um das Rohr (16) in Drehung versetzt werden;
**dadurch gekennzeichnet, dass** das Rührwerk peripher Magnetmassen (24) umfasst, um sie in der Nähe der Innenwand (8) der Wanne anzuordnen, wobei der Zubereiter ferner einen um die Innenwand (8) auf derselben Höhe wie die Magnetmassen (24) drehbaren Magnetring (23) umfasst, wobei die Magnete die Magnetmassen anziehen, wobei der Magnetring durch einen Motor (25) rotatorisch angetrieben wird, wobei der Zubereiter Mittel zur Erhitzung der Wand oder des Bodens der Wanne umfasst, um die Temperatur des Inhalts der Wanne zu regulieren.

2. Zubereiter eines Kulturmediums nach Anspruch 1, **dadurch gekennzeichnet, dass** er ferner einen ersten Heizhohlraum (9) in Kontakt mit der Innenwand (8) und um den zylindrischen Teil herum und einen zweiten Heizhohlraum (10) umfasst, der in Kontakt mit der Innenwand (8) und unter der Wanne angeordnet ist, wobei der Magnetring (23) in dem frei gelassenen Raum (22) zwischen den zwei Hohlräumen angeordnet ist.

3. Zubereiter eines Kulturmediums nach Anspruch 2, **dadurch gekennzeichnet, dass** jeder der Hohlräume (9, 10) mindestens ein Eingangsventil und ein Ausgangsventil umfasst, die es ermöglichen, einen den Hohlraum durchquerenden Fluidstrom zu erzeugen.

4. Zubereiter eines Kulturmediums nach einem der vorhergehenden Ansprüche, **dadurch gekennzeichnet, dass** das obere Ende des Rohrs (16) des Rührwerks durch eine Öffnung (18) des Deckels (3) verläuft, und dass das untere Ende des Rohrs (16) im Inneren eines Einsatzes (20) gleitet, der sich in der Mitte der halbkugelförmigen Kalotte befindet, wobei die Öffnung (18) des Deckels in der Vertikalen der Mittelachse der Wanne (3) angeordnet ist, wenn der Deckel abgesenkt wird, um den oberen Teil des Rührwerks zu zentrieren.

5. Zubereiter eines Kulturmediums nach Anspruch 4, **dadurch gekennzeichnet, dass** die im Wesentlichen halbkugelförmige Kalotte in ihrer Mitte mit einem Ablassloch durchbohrt ist, wobei das untere Ende des Rohrs einen Stopfen zum Verschließen des Rohrs darstellt.

6. Zubereiter eines Kulturmediums nach einem der vorhergehenden Ansprüche, **dadurch gekennzeichnet, dass** das Rohr (16) hohl und an seinen zwei Enden offen ist, um durch Ansaugen den flüssigen Inhalt aus der Wanne zu entnehmen.

7. Zubereiter eines Kulturmediums nach einem der vorhergehenden Ansprüche in Abhängigkeit von Anspruch 2, **dadurch gekennzeichnet, dass** er eine Regulierungsstelle (13) umfasst, die die Temperatur von Heizelementen, die in Kontakt mit den Hohlräumen (9, 10) angeordnet sind, kontrolliert, wobei die Regulierungsstelle (13) das Heizelement (12), das mit dem zweiten Hohlraum (10) in Kontakt angeordnet ist, nur dann aktiviert, wenn das im Inneren der Wanne angeordnete Produkt nicht über eine bestimmte Höhe hinausgeht.

## Claims

1. Culture medium preparator (1) comprising a cylindrical tank (2) that terminates at the low part in a substantially hemispherical cap, a lid (3) and a stirrer (15) placed in the tank (2), said stirrer comprising a vertical tube (16) placed in the center of the tank and a plurality of blades (17) put into rotation about said tube (16);
**characterized in that** the stirrer comprises magnetic masses (24) at the periphery so that they are placed in proximity to the internal wall (8) of the tank, the preparator furthermore comprising a magnetic ring (23) in rotation about the internal wall (8) at the same height as the magnetic masses (24), said magnets attracting said magnetic masses, the magnetic ring being moved in rotation by a motor (25), said preparator comprising means for heating the wall or the bottom of the tank to regulate in temperature the content of said tank.

2. Culture medium preparator according to claim 1, **characterized in that** it furthermore comprises a first heating cavity (9) in contact with the internal wall (8), and around the cylindrical wall and a second heating cavity (10) placed in contact with the internal wall (8) and beneath the tank, the magnetic ring (23) being placed in the space (22) left free between the two cavities.

3. Culture medium preparator according to claim 2, **characterized in that** each of the cavities (9, 10) comprises at least one inlet valve and one outlet valve enabling the creation of a current of fluid passing through said cavity.

4. Culture medium preparator according to any one of the above claims, **characterized in that** the upper extremity of the tube (16) of the stirrer passes through an orifice (18) in the lid and **in that** the lower extremity of the tube (16) slides into the interior of an insert (20) situated at the center of the hemispherical cap, the orifice (18) of the lid being situated vertically to the central axis of the tank (3) when the lid is lowered so as to center the upper part of the stirrer.

5. Culture medium preparator according to claim 4, **characterized in that** the substantially hemispherical cap is pierced at its center with a discharge hole, the lower extremity of the tube constituting a plug to block said hole.

6. Culture medium preparator according to any one of the above claims, **characterized in that** the tube (16) is hollow and open at both its extremities so as to extract the liquid content from the tank by suction.

7. Culture medium preparator according to any one of the above claims under the dependence of claim 2, **characterized in that** it comprises a control unit (13) controlling the temperature of heater elements placed in contact with the cavities (9, 10), said control unit (13) activating only the heater element (12) placed in contact with the second cavity (10) when the product placed inside the tank does not exceed a determined height.
